(19) Europäisches Patentamt
European Patent Office
Office européen des brevets

(11) EP 3 771 716 A1

(12) EUROPEAN PATENT APPLICATION

(43) Date of publication:
**03.02.2021 Bulletin 2021/05**

(21) Application number: **19460041.7**

(22) Date of filing: **02.08.2019**

(51) Int Cl.:
***C07D 487/04*** *(2006.01)* ***A61K 31/519*** *(2006.01)*
***A61P 35/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Zaklady Farmaceutyczne "Polpharma" S.A.**
**83-200 Starogard Gdanski (PL)**

(72) Inventors:
- **Nienaltowski, Tomasz**
  **83-200 Starogard Gdanski (PL)**
- **Pogoda, Dorota**
  **83-200 Starogard Gdanski (PL)**

(74) Representative: **Rechnio, Justyna Tatiana**
**Zaklady Farmaceutyczne Polpharma S.A.**
**Ul. Pelplinska 19**
**83-200 Starogard Gdanski (PL)**

# (54) LOW HYGROSCOPIC AMORPHOUS FORM OF BARICITINIB

(57) The invention relates to a low hygroscopic amorphous form of baricitinib and process for preparation thereof. It also refers to a pharmaceutical composition containing said amorphous form and to a use of said low hygroscopic amorphous form or pharmaceutical composition containing it for the treatment of rheumatoid arthritis.

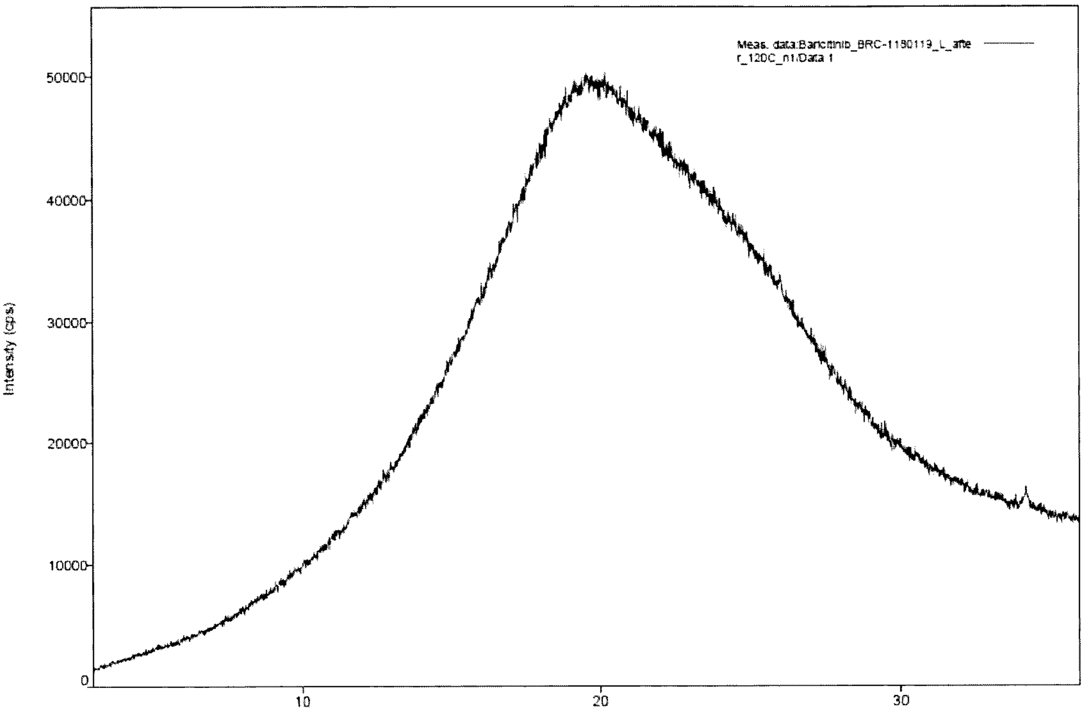


Figure 1: XRPD of low hygroscopic amorphous form of baricitinib

EP 3 771 716 A1

## Description

The invention relates to a low hygroscopic amorphous form of baricitinib and a process for preparation thereof. It also refers to pharmaceutical compositions containing said low hygroscopic amorphous form and to a use of said low hygroscopic amorphous form or pharmaceutical compositions for the treatment of rheumatoid arthritis.

### Background art

Baricitinib, i.e. {1-(Ethylsulfonyl)-3-[4-(7H-pyrrolo[2,3-d]pyrimidin-4-yl)-1H-pyrazol-1-yl]-3-azetidinyl}acetonitrile, has the following structural formula (1):

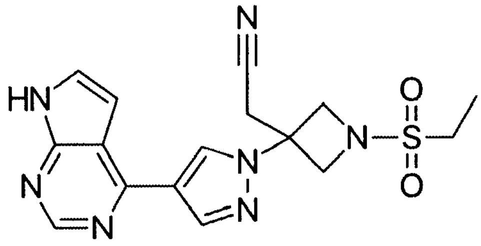

(1)

Baricitinib is an inhibitor of JAK1/JAK2 tyrosine kinase and is designated for treatment of rheumatoid arthritis.

WO2009/114512 A1 first discloses the process preparation of baricitinib free base as off-white solid (example 70).

WO2009/114512 A1 also discloses the process for preparation of baricitinib phosphate in a white crystalline form having a melting point of 187°C (example 71).

WO2009/114512 A1 further discloses the process for preparation of baricitinib trifluoroacetate in an unknown form (example 1).

WO2015/145286 relates to the amorphous form of baricitinib.

WO2015/166434, WO2016/141891, WO2019/003249 relates to crystalline forms of free base baricitinib.

There is a continuous demand in the pharmaceutical industry to be able to produce pure, morphologically homogenous and stable forms of active substances. Hygroscopicity of drug substances is an important parameter to be considered in product development. The moisture uptake rate varies for different drugs and excipients and is dependent on environmental conditions and it often affects stability of the drug in the dosage form. The impact of moisture on the chemical and physical stability of pharmaceuticals is of great concern, having significant implications for product quality, safety, and efficacy. Interaction of powders with water can result in a range of deleterious effects including caking and agglomeration of the powder, chemical instabilities that lead to degradation of active pharmaceutical ingredients (APIs), as well as changes in the dissolution profile of solid oral dosage forms. The effect of water on the properties of drug substances and on drug-excipient interactions has been reviewed, and moisture has been found to be implicated in inducing phase transformations, affecting chemical stability, and inducing changes in powder flow and mechanical properties during compaction.

Furthermore, from the point of view of the cost-effectiveness of production it is extremely important to produce the product using a procedure that may also be realized on the industrial scale that is easy to reproduce and results in a morphologically homogeneous, contaminant-free, and a stable form.

Amorphous form of baricitinib is known from WO2015145286, however the amorphous form obtained when following a process disclosed therein has the disadvantage of being significantly hygroscopic.

The hygroscopicity makes it difficult to handle the prior art amorphous form of baricitinib since costly and burdensome measures must be taken in order to ensure that the prior art amorphous form is not exposed to moisture during process and formulation.

Accordingly, the objective underlying the present invention was to produce a low hygroscopic amorphous form of baricitinib.

It has unexpectedly been found that it is possible to provide an amorphous form of baricitinib which is morphologically homogeneous, contaminant-free, stable and in particular has low hygroscopicity.

### Summary of the invention

The invention relates to a low hygroscopic amorphous form of baricitinib and a process for preparation thereof. It also refers to a pharmaceutical composition containing said low hygroscopic amorphous form and to a use of said low

hygroscopic amorphous form or pharmaceutical composition for the treatment of rheumatoid arthritis.

List of figures

Figure 1: shows a representative XRPD pattern of amorphous form of baricitinib.

Figure 2: shows a representative DSC plot of amorphous form of baricitinib.

Figure 3: shows a representative IR spectrum of amorphous form of baricitinib.

Detailed description of the invention

The present invention relates to a low hygroscopic amorphous form of baricitinib, having structural formula (1)

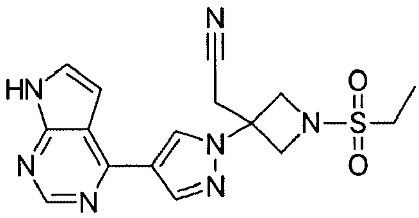

(1),

and provides a process for preparation of low hygroscopic amorphous form of baricitinib.

First aspect of the present invention is directed an amorphous form of baricitinib of low hygroscopicity, wherein said low hygroscopicity means a moisture content in the range of 0.3% to 0.6% w/w.

According to another embodiment of the first aspect, the present invention provides the amorphous form of baricitinib of low hygroscopicity wherein said low hygroscopicity means a moisture content in the range of 0.3% to 0.6% w/w after placing amorphous form of baricitinib for 24 hours in a desiccator maintained at a temperature of 40°C and 75±5% of relative humidity (RH).

According to another embodiment of the first aspect, the present invention provides amorphous form of baricitinib of low hygroscopicity wherein said low hygroscopicity means a moisture content of 0.6% w/w or less after placing amorphous form of baricitinib for 24 hours in a desiccator maintained at a temperature of 40°C and 75±5% relative humidity.

According to another embodiment of the first aspect, the present invention provides amorphous form of baricitinib of low hygroscopicity wherein said low hygroscopicity means a moisture content of 0.5% w/w or less after placing amorphous form of baricitinib for 24 hours in a desiccator maintained at a temperature of 40°C and 75±5% relative humidity.

According to another embodiment of the first aspect, the present invention provides amorphous form of baricitinib of low hygroscopicity wherein said low hygroscopicity means a moisture content of 0.4% w/w or less after placing amorphous form of baricitinib for 24 hours in a desiccator maintained at a temperature of 40°C and 75±5% relative humidity.

According to another embodiment of the first aspect, the present invention provides amorphous form of baricitinib of low hygroscopicity wherein said low hygroscopicity means a moisture content of 0.3% w/w or less after placing amorphous form of baricitinib for 24 hours in a desiccator maintained at a temperature of 40°C and 75±5% relative humidity.

According to another embodiment of the first aspect, the present invention provides low hygroscopic amorphous form of baricitinib wherein the said amorphous form has a powder X-ray diffractogram shown in Figure 1.

According to another embodiment of the first aspect, the present invention provides low hygroscopic amorphous form of baricitinib wherein said amorphous form reveals DSC thermogram with glass transition at 77±2°C and one exothermic peak with onset at 142±2°C and one endothermic peak with onset at 177±2°C.

According to another embodiment of the first aspect, the present invention provides low hygroscopic amorphous form of baricitinib wherein said amorphous form has an infrared spectrum comprising peaks at 3370 $cm^{-1}$, 3124 $cm^{-1}$, 1626 $cm^{-1}$, 1584 $cm^{-1}$, 1428 $cm^{-1}$, 1320 $cm^{-1}$, 1288 $cm^{-1}$, 1084 $cm^{-1}$, 897 $cm^{-1}$, 829 $cm^{-1}$ and 746 $cm^{-1}$.

According to another embodiment of the first aspect, the present invention provides low hygroscopic amorphous form of baricitinib wherein said amorphous form is substantially chemically pure, i.e. it contains less than 1 area-%, or

less than 0.5 area-%, preferably less than 0.1 area-%, of compounds other than baricitinib as determined by High Performance Liquid Chromatography(HPLC).

According to another embodiment of the first aspect, the present invention provides low hygroscopic amorphous form of baricitinib wherein said amorphous form does not substantially convert into a hydrated and/or crystalline form of baricitinib.

Second aspect of the present invention is related to a process for preparing low hygroscopic amorphous form of baricitinib as characterized above, wherein said process comprises the following steps:

(i) dissolving baricitinib free base in an organic solvent;

(ii) adding antisolvent to the solution obtained in step (i) to precipitate amorphous form of baricitinib

(iii) collecting the resultant low hygroscopic amorphous form of baricitinib.

It has been found that this process provides low hygroscopic amorphous form of baricitinib with a high yield and high purity.

In step (i) of the above process, the baricitinib free base is dissolved in an organic solvent. Suitable solvents include tetrahydrofuran, 2-methyltetrahydrofuran, acetonitrile, dimethylacetamide and/or acetone. The most preferred solvent is tetrahydrofuran.

To effect dissolution, the solvent is preferably heated, and most preferably the solvent is heated under reflux.

In step (ii), the antisolvent is preferably miscible with the solvent and baricitinib must be poorly soluble in antisolvent. Suitable antisolvents include ethyl acetate, isopropyl acetate, methanol, n-heptane, cyclohexane, toluene and/or water. Most preferably the antisolvent is n-heptane.

To effect precipitation, the antisolvent is preferably added to the solution obtained in step (i) at 25-45°C, and most preferably at 30-35°C.

In step (iii), the resultant amorphous form of baricitinib is preferably collected by filtration. It is then typically washed with the antisolvent(s) and dried.

According to another embodiment of the second aspect, the present invention provides another process for the preparation of low hygroscopic amorphous form of baricitinib comprising:

(i) dissolving baricitinib free base in an organic solvent or a mixture thereof;

(ii) removing the solvent from the solution obtained in step (i) by suitable technique to provide low hygroscopic amorphous form of baricitinib.

In step (i) of the above process, the baricitinib free base is dissolved in an organic solvent. Suitable solvents include tetrahydrofuran, 2-methyltetrahydrofuran, acetonitrile, dimethylacetamide and/or acetone. The solvent is most preferably acetone.

To effect dissolution, the solvent is preferably heated, and most preferably the solvent is heated under reflux.

In step (ii) of the present process, the suitable technique that may be used for the removal of solvent from the solution includes but is not limited to evaporation, evaporation under reduced pressure, flash evaporation, vacuum drying, concentrating a solution, distillation under atmospheric pressure, vacuum distillation, distillation by using a rotational distillation device such as a Buchi rotavapor, agitated thin film drying (ATFD), melt extrusion, spray drying, freeze drying (lyophilization), spray-freeze drying or by any other suitable technique.

The solvent may be removed at temperatures ranging from 25°C to 100°C, optionally under reduced pressure.

Further object of the invention is a pharmaceutical composition comprising low hygroscopic amorphous form of baricitinib and at least one pharmaceutically acceptable excipient.

In the preferred embodiment of the above, the pharmaceutical composition according to the present invention is formulated into a solid unit dosage form for oral administration, preferably into a tablet.

Exemplary pharmaceutically acceptable excipients which can be contemplated for use in the pharmaceutical composition of the present invention are described below.

Fillers are excipients which increase the volume of e.g. tablets. Examples of fillers are lactose, mannitol, celluloses such as microcrystalline cellulose, synthetic polymers, Ca-phosphate, inorganic calcium salts, maize starch, polyols and pregelatinized starch.

Binding agents are excipients which increase the adhesion of the active agents and excipients during granulation. Examples of binding agents are: sugars, such as sorbitol, glucose, fructose, disaccharides as saccharose, polysaccharides; acacia gum; cellulose derivatives, such as soluble celluloses like methylcellulose, hydroxypropyl methylcellulose and hydroxypropyl cellulose; tragacanth; polyvinylpyrrolidone, such as N-vinylpyrrolidone or polyvinylpyrrolidonevinyl acetate copolymer; sodium alginate and alginate derivatives and gelatin.

Disintegrants are excipients which can take up water and swell and thus improve disintegration of a tablet or granules. Examples of disintegrants are: croscarmellose sodium; starch (paste and pre-gelatinized), such as sodium starch glycolate, croscarmellose sodium and low substituted hydroxypropyl cellulose, methacrylic acid polymer with divinylbenzene, potassium salt, maltodextrin; and crospovidone.

Lubricants are excipients which reduce the friction between excipients and compression tooling surfaces. Examples of lubricants are magnesium stearate, magnesium fumarate, fumaric acid, and sodium stearyl fumarate.

A polymer, or a plurality of polymers may be used to coat the solid dosage form, if necessary, and they include cellulose derivatives, e.g. hydroxypropyl methylcellulose, polyvinylpyrrolidones, polyethylene glycols, polyvinyl alcohols, acrylates, such as polymethacrylate, cyclodextrins and copolymers and derivatives thereof, including for example polyvinylpyrolidine-vinylacetate. In some preferred embodiments the polymer or the plurality of polymers are pH dependent enteric polymers. Such polymers include cellulose derivatives, e.g. cellulose acetate phthalate, hydroxypropyl methyl cellulose phthalates, hydroxypropyl methyl acetate succinate, hydroxypropyl methyl cellulose acetate, carboxymethylcellulose or a salt thereof, e.g. the sodium salt, cellulose acetate trimellitate, hydroxypropyl cellulose acetate phthalate, or polymethyl acrylates, e.g. Eudragit®S.

Surfactants include but are not limited to, sorbitan fatty esters, polyoxymethylene sorbit esters, sodium lauryl sulfate, sodium docedylbenzenesulfate, dioctyl sodium sulfosuccinate, sodium stearate, EDTA or vitamin E or tocopherol derivatives.

Other excipients are known in the art and can be chosen by a skilled person depending on their function. Reference may be made in this context to Handbook of Pharmaceutical Excipients, ed. by R.C. Rowe et al, 6th edition, Pharmaceutical Press (2009).

Formulating the low hygroscopic amorphous form of baricitinib into a pharmaceutical dosage form may be carried out by applying techniques and excipients known in the art. Reference is made herein for example to Pharmaceutical Manufacturing Handbook; Production and Processes, ed. S.C. Gad; John Wiley and Sons, (2008).

Another object of the present invention is a low hygroscopic amorphous form of baricitinib, or a pharmaceutical composition comprising low hygroscopic amorphous form of baricitinib for the treatment of rheumatoid arthritis.

Experimental part

Measuring instruments, conditions and protocols:

Within the meaning of the present invention, the term "room temperature" as used herein is understood to mean temperatures of about 15°C to about 25°C.

HPLC

Purity and assay analysis were carried out using High Performance Liquid Chromatograph equipped with PDA detector. The analysis was conducted using Octadecyl silane (ODA or C18) chemically bonded to porous silica column, at a temperature of 40°C. The mobile phase was acetonitrile and 0.1% (v/v) ortho-phosphoric acid in water used in gradient.

KF

Water content in samples of baricitinib was determined using Mettler Toledo V30 Karl Fisher Titrator. The analysis were carried out using acetonitrile: methanol (50:50) solution and the Combi Titrant 2 Merck titrant.

XRPD

Powder X-ray diffraction was performed with a Rigaku MiniFlex 600 diffractometer with Bragg-Brentano geometry CuKa radiation and D/teX Ultra Detector. The powder X-ray diffraction patterns were recorded at a tube voltage of 40kV and a tube current of 15 mA, applying a step size of 0.01° and speed 6.0°/min with the angular range 3-36° at ambient conditions.

DSC

DSC analysis was carried out using DSC 1 Mettler Toledo Stare thermal analysis system in the temperature range of 30-250°C with the gradient of 10°C/min. Aluminum crucible with the capacity of 40$\mu$l were used. Nitrogen was used as carried gas with the flow rate of 40ml/min. The accuracy of the measurements is $\pm$2°C

IR measurements

IR analysis were performed on FT-IR Thermo Nicolet Avatar 370 spectrometer in ATR method and PIKE TECHNOLOGIES Smart MIRacle TM single Reflection HATR module. The accuracy of the measurements is ±2 $cm^{-1}$.

Hygroscopicity measurment

In this method, moisture sorption has been measured gravimetrically by placing a pre-weighed material in a closed desiccator (humidity chamber), which contains a well filled with saturated solution of ammonium chloride salt. A calibrated thermohygrometer is placed inside desiccator for monitoring temperature and humidity. About 300-500 mg of each sample is transferred into dry Petri-dish and kept in the desiccator maintained at 40°C, 75±5% relative humidity. After keeping for 24 h, the samples were removed from the desiccator, and the final weight of each sample was determined with the help of a calibrated balance to check the moisture sorption and samples were reanalyzed by XRPD to check the stability of samples.

The percentage weight gain of each sample was calculated using below equation

$$W = W2 - W1 / W1 \times 100$$

where,

W = total weight gain
W1 = initial weight of the material before loading into desiccator
W2 = weight of the sample after exposing to 40°C, 75±5% relative humidity in the desiccator.

The following examples describe the present invention in detail, but are not to be construed to limit the present invention.

Example 1 - Preparation of 4-(1H-pyrazol-4-yl)-7-((2-(trimethylsilil)ethoxy)methyl)-7H-pyrrolo[2,3-d]pyrimidine (6)

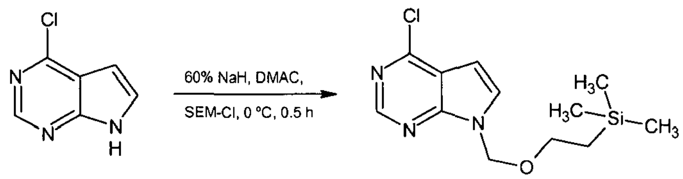


2 3

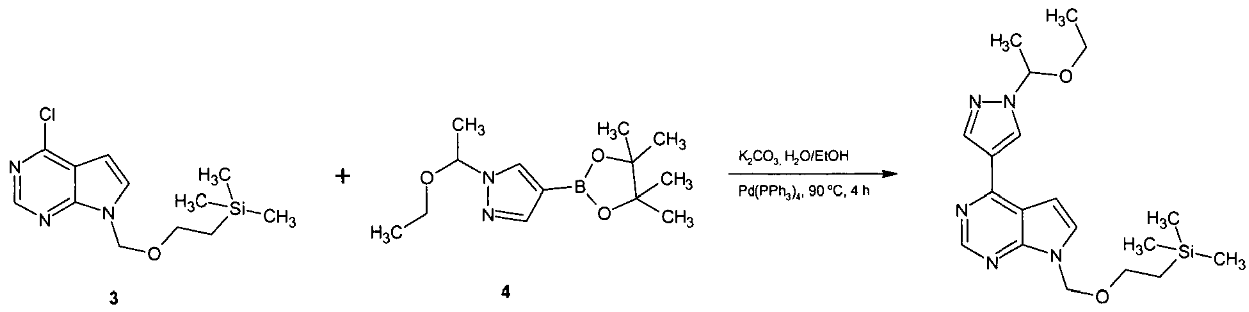


3 4 5

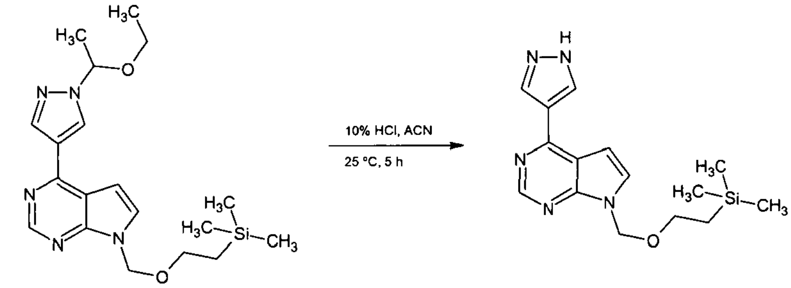


5 6

To a mixture of sodium hydride (7.15 g, 0.1797 mol, 1.1 eq) in N,N,-dimethylacetamide (100 ml), 4-chloro-7H -pyrrolo[2,3-d]pyrimidine (25 g, 0.1628 mol, 1 eq) in N,N,-dimethylacetamide (150 ml) is added under stirring while maintaining the temperature of reaction mass at 0-5°C. The resulting reaction mass is stirred for 30 minutes at 0-5°C. SEM chloride (trimethylsilyl ethoxymethyl chloride) (30 g, 0.1797 mol, 1.1 eq) is then added dropwise to the reaction mixture while maintaining the temperature about 0-5°C. The reaction mixture is stirred at 0-5°C for 1-2 hours. After the completion of the reaction, the reaction mass is quenched with water (50 ml). The reaction mixture is diluted with additional water (200 ml) and is extracted with ethyl acetate (250 ml). The aqueous layer is extracted twice with ethyl acetate (2 x 250 ml). The combined organic layers are washed with saturated brine solution (125 ml) and with water (250 ml). The organic layer is concentrated at 40°C under vacuum to obtain 4-chloro-7-[2-(trimethylsilyl)ethoxy]methyl-7H-pyrrolo[2,3-d]pyrimidine (3).

To 4-chloro-7-[2-(trimethylsilyl)ethoxy]methyl-7H-pyrrolo[2,3-d]pyrimidine (3) a mixture of solvent: (water 115.5 ml) and ethanol (347 ml), potassium carbonate (50.6 g, 0.3663 mol, 2.25 eq), 1-(1-ethoxyethyl)-4-(4,4,5,5-tetramethyl-1,3,2-dioxaborolan-2yl)-1H-pyrazole (45.5 g, 0.1710 mol, 1.05 eq) is added at room temperature. Resulting reaction mixture is degassed with nitrogen for 20 min and terakis(triphenylphosphine)palladium(0) (0.38 g, 0.000329 mol, 0.002 eq) is added as catalyst at room temperature. The reaction mixture is kept under gentle reflux at 85-90°C for 2 hrs. After the completion of reaction, the reaction mixture is cooled to room temperature. The suspension is filtered and washed with ethyl acetate (225 ml). Layers are separated and the organic layer is concentrated under reduced pressure at 45°C to obtain 4-(1-(1-ethoxyethyl)-1H-pyrazol-4-yl)-7-((2-(trimethylsilyl)ethoxy)methyl)-7H-pyrrolo[2,3-d]pyrimidine (5).

To 4-(1-(1-ethoxyethyl)-1H-pyrazol-4-yl)-7-((2-(trimethylsilyl)ethoxy)methyl)-7H-pyrrolo[2,3-d]pyrimidine(5) acetonitrile (50 ml) and water (135 ml) are added. Reaction mixture is heated to 35-45°C and 10% aqueous HCl solution is added at room temperature to adjust the pH below 0.5. Resulting reaction mixture is stirred at 35-45°C for 2 h. After the completion of reaction, reaction mixture is cooled to 20-30°C. The solid is collected by filtration, washed with water (250 ml) and dried at reduced pressure at 50-60°C to obtain [4-(1H-pyrazol-4-yl)-7-((2-(trimethylsilyl)ethoxy)methyl)-7H-pyrrolo[2,3-d]pyrimidine(6).
Yield: 41.6 - 45.7 g (80-90%)
HPLC Purity: 98-99%

<u>Example 2 -Preparation of 2-(1-(ethylsulfonyl)-3-(4-(7-((2-(trimethylsilyl)ethoxy)methyl)-7H-pyrrolo[2,3-d]pyrymidin-4-yl)-1H-pyrazol-1-yl)azetidin-3-yl)acetonitrile (8)</u>

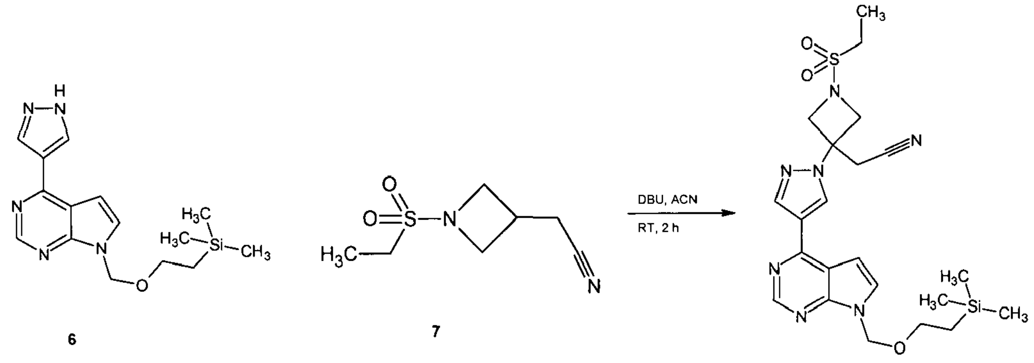


6 7 8

[4-(1H-pyrazol-4-yl)-7-((2-(trimethylsilyl)ethoxy)methyl)-7H-pyrrolo[2,3-d]pyrimidine(6) (40.0 g, 0.1268 mol, 1.0 eq) and [1-(ethylsulfonyl)azetidin-3-yl idene]acetonitrile (7) (22.4 g, 0.1205 mol, 0.95 eq) are added to a reactor containing acetonitrile (300 ml). DBU (19.3 g,0.1268 mol, 1.0 eq) is slowly added at 20-30°C. Reaction mass is stirred for 2 h at 20-30°C. After completion of the reaction, water (1000 ml) is added to the reaction mass and stirred for 2 hours at room temperature. The solid is filtered, washed with water (400 ml) and methyl tert-butyl ether(200 ml), and dried at 50°C in an air dryer for 4-5 hrs. to obtain 2-(1-(ethylsulfonyl)-3-(4-(7-((2-(trimethylsilyl)ethoxy)methyl)-7H-pyrrolo[2,3-d]pyrymidin-4-yl)-1H-pyrazol-1-yl)azetidin-3-yl)acetonitrile (8).
Yield: 48.9 - 54.1 g (76 - 86%)
HPLC Purity: 98-99%

<u>Example 3 -Preparation of crude baricitinib (1)</u>

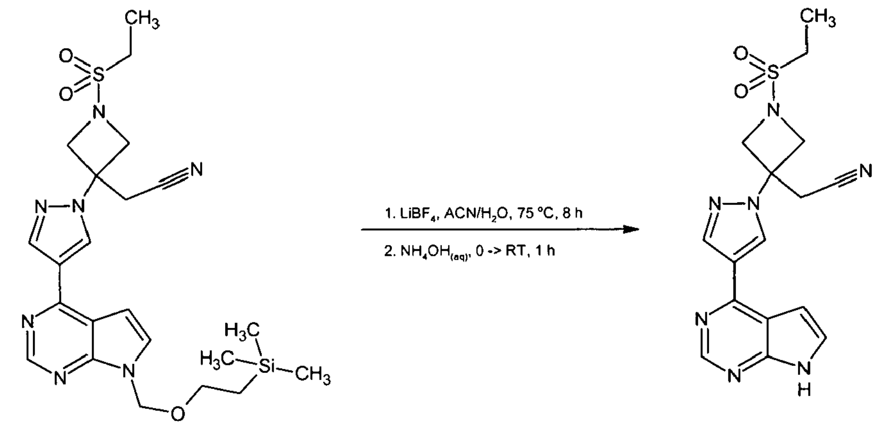


8 1

To a solution of 2-(1-(ethylsulfonyl)-3-(4-(7-((2-(trimethylsilyl)ethoxy)methyl)-7H-pyrrolo[2,3-d]pyrymidin-4-yl)-1H-pyrazol-1-yl)azetidin-3-yl)acetonitrile (8) (50 g, 0.0996 mol, 1.0 eq) in acetonitrile (450 ml) and water (45 ml), lithium tetrafluoroborate (102.7 g, 1.0963 mol, 11 eq) is added at room temperature (20-30°C). The reaction mixture is heated to 80-90°C and stirred for 12 hours. After the completion of reaction, the reaction mixture is cooled to 0-10°C, solution of ammonium hydroxide 25% is added slowly into the reaction mixture to adjust the pH to 9-10. The reaction mixture is then stirred for 1 hour at room temperature. Next, ethanol (500 ml) is added to reaction mixture, followed by heating at 50-60°C. The reaction mixture is stirred for 1 h at 50-60°C, filtered and washed with ethanol (150 ml). The filtrate is distilled out under reduced pressure (500 ml), water is added (500 ml) and the organic solvent is removed again under reduced pressure (250 ml). Residue after distillation is gradually cooled to 0-10°C and stirred for 2 h at 0-10°C. Solid is filtered and washed with water (250 ml) and dried at 45°C under vacuum to obtain crude baricitinib free base (1).
Yield: 29.9 - 33.1 g (80 - 90%)
HPLC Purity: 99.0-100%

Example 4 -Preparation of baricitinib (1).

Crude baricitinib free base of Example 3 (30 g, 0.0807 mol, 1.0 eq) is suspended in an ethanol/water mixture 3:1 (450 ml) and reaction mass is heated to 70-80°C to obtain clear solution. Activated carbon (3 g) is added followed by stirring for 1 h at 70-80°C. Suspension is filtered and washed with ethanol/water mixture 3:1 (60 ml). Filtrate is extracted with n-heptane and then gradually cooled down to 0-10°C within 4 h and stirred for another 2 h. Solid is filtered off, washed with water (45 ml) and obtained solid is dried at 45°C under vacuum 8-10 hrs. to provide baricitinib (1).
Yield: 24.3 - 27.3 g (81 - 91%)
Purity: 99.5-100%

Example 5 -Preparation of low hygroscopic amorphous form of baricitinib.

Baricitinib obtained in example 4 (20 g), tetrahydrofuran (100 ml) are added to a reaction vessel at room temperature. The reaction mixture is heated to reflux to dissolve baricitinib. The reaction mixture is cooled to 30-35°C and n-heptane (250 ml) is added dropwise to precipitate the solid. The reaction mass is cooled to 0-5°C and maintained for 3 hours. The solid is filtered off and dried under vacuum at 40-45°C to yield low hygroscopic amorphous form of baricitinib.
Yield : 90%
Water content (K.F.) - 0.42%

Example 6 -Preparation of low hygroscopic amorphous form of baricitinib.

Baricitinib obtained in Example 4 (20 g), dimethyl acetamide (100 ml) are added to a reaction vessel at room temperature. The reaction mixture is heated to reflux to dissolve baricitinib. The reaction mixture is cooled to 30-35°C and water (250 ml) is added dropwise to precipitate the solid. The reaction mass is cooled to 0-5°C and maintained for 3 hours. The solid is filtered off and dried under vacuum at 40-45°C to yield low hygroscopic amorphous form of baricitinib.
Yield : 90%
Water content (K.F.) - 0.35%

Example 7 -Preparation of low hygroscopic amorphous form of baricitinib.

Baricitinib obtained in Example 4 (20 g), acetone (200 ml) are added to a reaction vessel at room temperature. The reaction mixture is heated to reflux to dissolve baricitinib. The reaction mixture is cooled to 40-45°C and the solvent is removed by distillation under vacuum. The obtained solid is dried under vacuum at 40°C to obtain low hygroscopic amorphous form of baricitinib.
Yield : 95%
Water content (K.F.) - 0.40 %

Example 8 -Preparation of low hygroscopic amorphous form of baricitinib.

Baricitinib obtained in Example 4 (20 g) is dried at 100-120°C in an air dryer to obtain low hygroscopic amorphous form of baricitinib.
Yield : 98%
Water content (K.F.) - 0.45%

Comparative example 1 - WO2015145286 -Preparation of amorphous form of baricitinib.

4-(1-(3-cyanomethyl)-1-(ethylsulfonyl)azetidine-3-yl)-1H-pyrazol-4-yl)-7H-pyrrolo[2,3-d]pyrimidin-7-yl)methyl pivalate (1g), methanol (5ml), tetrahydrofuran(20 ml), and 1M sodium hydroxide(2.3ml) were added into a reaction vessel at 20°C to 25°C. The reaction mixture was stirred for 3 hours. Progress of the reaction was monitored by thin layer chromatography. On completion, the reaction mixture was quenched by adding water (20 ml). The pH was adjusted to 7.0 to 7.5 by adding 1N HCl, followed by completely recovering the solvent under reduced pressure at 40 to 50°C. A sticky material was obtained. Water (10 ml) was added to the sticky material at 20 to 25°C. The contents were stirred for 10 minutes. A sold material was precipitated out. The solid material was filtered, washed with water (20ml), and then dried under reduced pressure at 40 to 45°C for 24 hours to obtain the amorphous form of baricitinib.
Yield : 81%
Water content (K.F.) - 1.6%

Example 9 - Hygroscopicity of amorphous form of baricitinib.

The samples of amorphous form of baricitinib obtained from example 5, example 6, example 7, example 8 and comparative example 1 were stored in a humidity chamber (closed desiccator) at 40°C, 75±5% relative humidity for 24 hours, relative humidity and visual observation was performed at regular intervals. The results are given in Table 1 and 2.

Table 1.

| Examples | Conditions | T = 2 h | T = 6 h | T = 12 h | T = 24 h |
|---|---|---|---|---|---|
| Example 5 | 40°C, 75±5% relative humidity | No deliquescence | No deliquescence | No deliquescence | No deliquescence |
| Example 6 | | No deliquescence | No deliquescence | No deliquescence | No deliquescence |
| Example 7 | | No deliquescence | No Deliquescence | No deliquescence | No deliquescence |
| Example 8 | | No deliquescence | No Deliquescence | No deliquescence | No deliquescence |
| Comparative example 1 | | No deliquescence | Sticky solid | Partial deliquescence | deliquescence |

After keeping for 24 h, the samples were removed from the desiccator, and the final weight of each sample was determined using a calibrated balance to check the moisture sorption and samples were reanalyzed by XRPD to check the stability of samples. The percentage weight gain of each sample was calculated using below equation

$$W = W2\text{-}W1 / W1 \times 100$$

where,

W = total weight gain

W1 = initial weight of the material before loading into desiccator

W2 = weight of the sample after exposing to 40°C, 75±5% relative humidity in the desiccator.

Table 2 : Percentage weight gain

| Conditions | Percentage weight gain (%) | | | | |
|---|---|---|---|---|---|
| | Example 5 | Example 6 | Example 7 | Example 8 | Comparative example 1 |
| 40°C, 75±5 % relative humidity | 0.42% | 0.36% | 0.40% | 0.45% | 4.56% |

Result shows amorphous form obtained in Examples 5, 6, 7 and 8 are non-hygroscopic as compared to amorphous form obtained in Comparative example 1 after exposing to 40°C, 75±5% relative humidity in the desiccator for 24 hours.

The X-ray diffractogram of amorphous form obtained in Example 5, 6, 7 and 8 showed no substantial change during the course of the experiment, meaning that no change in the form was obtained. This is in contrast to amorphous form obtained in Comparative example 1, which deliquesced within 24 hours upon storage at 40°C and 75±5% relative humidity.

## Claims

1. Low hygroscopic amorphous form of baricitinib, wherein the moisture content is in the range of 0.3% to 0.6% w/w.

**2.** Low hygroscopic amorphous form of baricitinib according to claim 1, wherein powder X-ray diffractogram is substantially the same as shown in Figure 1.

**3.** Low hygroscopic amorphous form of baricitinib according to claim 1 or 2, **characterized by** DSC thermogram with glass transition at 77±2°C and one exothermic peak with onset at 142 ± 2°C and one endothermic peak with onset at 177±2°C.

**4.** Low hygroscopic amorphous form of baricitinib according to any of claims 1 to 3, **characterized by** infrared spectrum comprising peaks at 3370 $cm^{-1}$, 3124.60 $cm^{-1}$, 1626 $cm^{-1}$, 1584 $cm^{-1}$, 1428 $cm^{-1}$, 1320 $cm^{-1}$, 1288 $cm^{-1}$, 1084 $cm^{-1}$, 897 $cm^{-1}$, 829 $cm^{-1}$ and 746 $cm^{-1}$.

**5.** Low hygroscopic amorphous form of baricitinib according to any of claims 1 to 4, wherein the moisture content in the range of 0.3% to 0.6% w/w after placing amorphous form of baricitinib for 24 hours in a desiccator maintained at a temperature of 40°C and a humidity level of 75±5%.

**6.** Low hygroscopic amorphous form of baricitinib according to claim 5 wherein the moisture content of 0.6% w/w or less after placing amorphous form of baricitinib for 24 hours in a desiccator maintained at a temperature of 40°C and a humidity level of 75±5%.

**7.** A process for preparing low hygroscopic amorphous form of baricitinib, wherein said process comprises the following steps:

(i) dissolving baricitinib free base in an organic solvent;
(ii) adding antisolvent to the solution obtained in step (i) to precipitate amorphous form of baricitinib
(iii) collecting the resultant low hygroscopic amorphous form of baricitinib .

**8.** The process according to claim 7, wherein baricitinib free base is dissolved in a solvent selected from tetrahydrofuran, 2-methyltetrahydrofuran, acetonitrile, dimethylacetamide and/or acetone.

**9.** The process according to claim 7 or 8, wherein the antisolvent is selected from ethyl acetate, isopropyl acetate, methanol, n-heptane, cyclohexane, toluene and/or water.

**10.** A process for preparing low hygroscopic amorphous form of baricitinib, wherein said process comprises the following steps:

(i) dissolving baricitinib free base in an organic solvent or a mixture of organic solvents;
(ii) removing the solvent from the solution obtained in step (i) by suitable technique to provide low hygroscopic amorphous form of baricitinib.

**11.** The process according to claim 10, wherein baricitinib free base is dissolved in a solvent selected from tetrahydrofuran, 2-methyltetrahydrofuran, acetonitrile, dimethylacetamide and/or acetone.

**12.** The process according to claim 10 or 11, wherein the suitable technique for removing solvent is evaporation, evaporation under reduced pressure, flash evaporation, vacuum drying, concentrating a solution, atmospheric distillation, vacuum distillation, distillation by using a rotational distillation device such as a Buchi rotavapor, agitated thin film drying, melt extrusion, spray drying, freeze drying and /or spray-freeze drying.

**13.** Pharmaceutical composition comprising the low hygroscopic amorphous form of baricitinib of any of claims 1-6 and at least one pharmaceutically acceptable excipient.

**14.** The pharmaceutical composition according to claim 13, wherein said pharmaceutical composition is formulated into a solid unit dosage form for oral administration, preferably into a tablet.

**15.** The low hygroscopic amorphous form of baricitinib of any of claims 1-6 or the pharmaceutical composition of claims 13-14 for the treatment of rheumatoid arthritis.

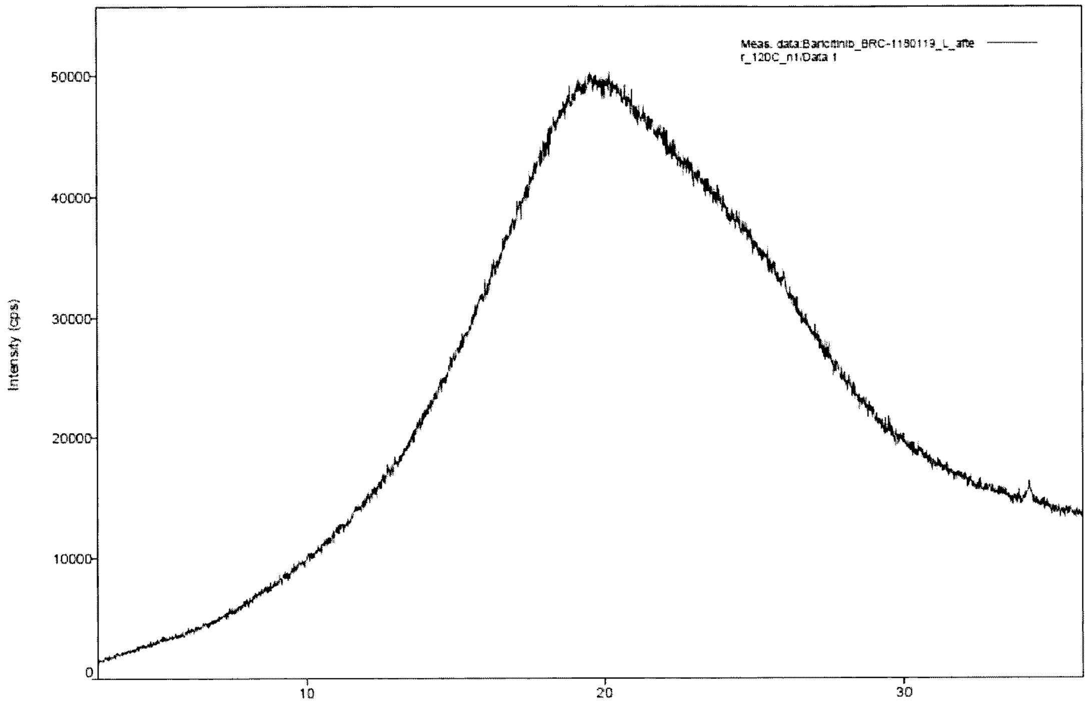


Figure 1: XRPD of low hygroscopic amorphous form of baricitinib

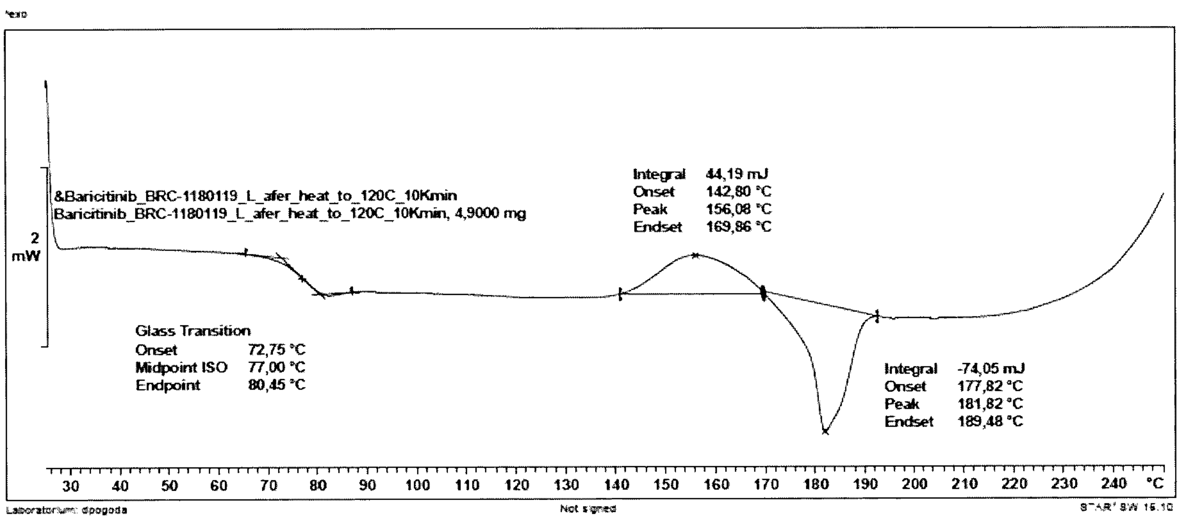


Figure 2: DSC of low hygroscopic amorphous form of baricitinib

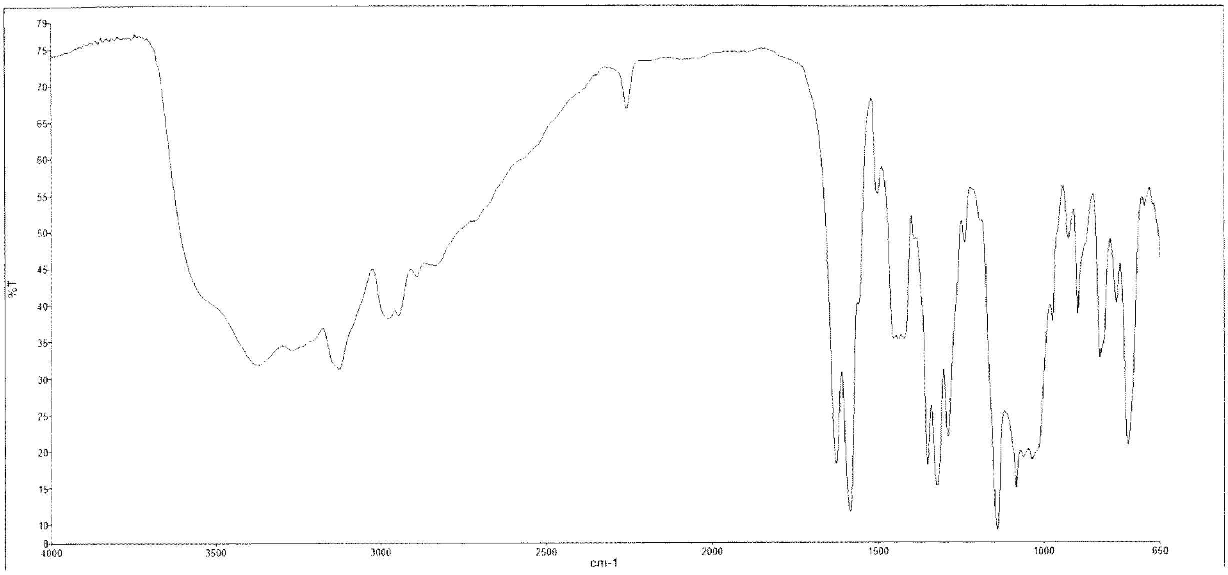


Figure 3: Infrared spectrum of low hygroscopic amorphous form of baricitinib

Europäisches Patentamt
European Patent Office
Office européen des brevets

# EUROPEAN SEARCH REPORT

Application Number
EP 19 46 0041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2015/145286 A1 (SUN PHARMACEUTICAL IND LTD [IN]) 1 October 2015 (2015-10-01)<br>* claims; examples *<br>----- | 7-12 | INV.<br>C07D487/04<br>A61K31/519<br>A61P35/00 |

TECHNICAL FIELDS SEARCHED (IPC)

C07D

The present search report has been drawn up for all claims

1

| Place of search | Date of completion of the search | Examiner |
|---|---|---|
| Berlin | 11 February 2020 | Beyss-Kahana, Ellen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.........................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 46 0041

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2020

| Patent document cited in search report | Publication date | Patent family member(s) | Publication date |
|---|---|---|---|
| WO 2015145286 A1 | 01-10-2015 | EP 3134412 A1 | 01-03-2017 |
| | | US 2017129895 A1 | 11-05-2017 |
| | | WO 2015145286 A1 | 01-10-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009114512 A1
- WO 2015145286 A
- WO 2015166434 A
- WO 2016141891 A
- WO 2019003249 A


### Non-patent literature cited in the description


- Handbook of Pharmaceutical Excipients. Pharmaceutical Press, 2009
- Pharmaceutical Manufacturing Handbook; Production and Processes. John Wiley and Sons, 2008